# EUROPEAN PATENT SPECIFICATION

(11) **EP 4 223 250 B1**
(45) Date of publication and mention of the grant of the patent: **04.11.2026**
(21) Application number: 23154784.5
(22) Date of filing: 02.02.2023
(51) Int. Cl.: G02B 21/00, G02B 21/36, A61B 90/20, G02B 21/24, G06N 20/00, G06T 7/73, G06V 10/25, G06V 20/69, G06V 40/18, A61B 90/00

(54) **SURGICAL MICROSCOPE SYSTEM AND SYSTEM, METHOD AND COMPUTER PROGRAM FOR A MICROSCOPE OF A SURGICAL MICROSCOPE SYSTEM**
OPERATIONSMIKROSKOPSYSTEM UND SYSTEM, VERFAHREN UND COMPUTERPROGRAMM FÜR EIN MIKROSKOP EINES OPERATIONSMIKROSKOPSYSTEMS
SYSTÈME ET SYSTÈME DE MICROSCOPE CHIRURGICAL, PROCÉDÉ ET PROGRAMME INFORMATIQUE POUR UN MICROSCOPE D'UN SYSTÈME DE MICROSCOPE CHIRURGICAL

(30) Priority: 08.02.2022 DE 102022102898
(43) Date of publication of application: 09.08.2023
(73) Proprietor: Leica Instruments (Singapore) Pte Ltd, Singapore 608924 (SG)
(72) Inventor: THEMELIS, George, Singapore 608924 (SG)
(74) Representative: 2SPL Patentanwälte PartG mbB

(56) References cited:
- EP-A1- 3 806 037
- WO-A1-2018/076094
- US-A1- 2019 324 252
- US-B1- 10 758 309

## Description

### Technical field

Examples relate to a surgical microscope system and to a system, a method, and a computer program for a microscope of a surgical microscope system.

### Background

Autofocus is a functionality that is commonly used in optical cameras today. For example, surgical microscopes often provide an autofocus functionality. However, in surgical microscopes, the usual autofocus function typically uses a predefined image area, which is used as a focus reference. In many cases, the area of interest to the surgeon is often not within this autofocus area. This can lead the camera to focus on areas other than the surgeon intended.

In smartphones and mirrorless interchangeable lens cameras, an enhanced autofocus functionality is provided that searches for human faces and attempts to focus on the faces. Even when the faces move within the image, the autofocus may keep searching for the faces and adjust the focus accordingly.

In surgical microscopes and other medical optical imaging devices, the area of interest is not as universal as a human face. Instead, it could be anything, such as an anatomical or morphological structure, e.g., a vessel or a surgical cavity, respectively. Thus, it is not possible to predefine a spatial pattern, such as a face, and search for it within the image to set the autofocus target.

WO 2018/076094 A1 discloses a method for focusing on a viewing target. The viewing target can be dynamically defined by the surgeon. For example, the surgeon may adjust the viewing target using a user interface provided by a workstation, by touching the desired target on a touch-sensitive display, by using eye or head tracking to detect a point at which the surgeon's gaze is focused and/or by voice command. The imaging system may perform autofocusing based on the viewing target.

US 2019/324252 A1 discloses a surgical microscope system for use in a medical procedure. The surgical microscope system is controlled in a first mode of operation corresponding to a phase of the medical procedure and defining at least one setting for adjusting a set of adjustable optics. While in the first mode of operation, the surgical microscope system automatically determines, from a captured image, an indication that a second mode of operation is relevant. The mode of operation is switched to the second mode of operation.

There may be a desire for an improved autofocus functionality for a surgical microscope.

### Summary

This desire is addressed by the subject-matter of the independent claims.

The proposed concept is based on the finding, that, during surgery, the surgeon concentrates on a small region (i.e., the actual surgical site), with other portions around that area being of less interest. This region is defined or delineated by one or a few anatomical features, with the surgeon either operating on these anatomical features or between these anatomical features. In the proposed concept, the surgeon may point to an area in the image and, doing so, trigger the proposed functionality, to make sure that the image focus is automatically focused to this area. The surgeon may provide instructions that identify the anatomical feature in this area that needs to be followed, e.g., the system may be instructed to follow a user-specified vessel in a given area, or the instructions may be unspecific so that the proposed functionality may make some assumptions about which of the features are implied by the area indicated, e.g., to select a surgical cavity if the area indicated by the surgeon contains a surgical cavity. The proposed concept may thus allow the surgeon to select the autofocus area in a smart and meaningful way, such that the autofocus follows a certain image feature, instead of a spatially fixed area of the image.

Various examples of the present disclosure relate to a system for a microscope of a surgical microscope system. The system comprises one or more processors and one or more storage devices. The system is configured to obtain imaging sensor data from at least one optical imaging sensor of the microscope. The system is configured to determine information on an area of interest of a user of the surgical microscope system based on an input of the user. The system is configured to determine an anatomical feature of interest within the area of interest. The system is configured to detect a position of the anatomical feature of interest within the imaging sensor data. The system is configured to trigger an autofocus functionality of the microscope to focus on the position of the anatomical feature of interest. By determining an area of interest based on a user input, a detection algorithm may be provided with sufficient information for determining the likely feature of interest, which may be tracked and used as target for the autofocus functionality, such that the likely feature of interest remains in focus throughout the surgical procedure.

In general, the system is configured to track the position of the anatomical feature of interest over a plurality of frames of the imaging sensor data. By tracking the feature over multiple frames, the autofocus functionality may be triggered to adjust the focus based on the current position of the anatomical feature of interest.

For example, the system may be configured to trigger the autofocus functionality if the position of the anatomical feature of interest shifts relative to a field of view of the imaging sensor data for at least a pre-defined time interval. In other words, if the position of the feature of interest is shifted not only temporarily (e.g., as the field of view is changed by the surgeon, or as the surgeon has moved the feature of interest), the autofocus functionality may be employed to adjust the focus to the current position of the anatomical feature of interest.

While surgical microscope systems are generally equipped with powerful processors for image processing, the efficiency of the proposed concept may be improved by limiting the detection of the position of the anatomical feature to only a subset of the frame, e.g., to every n-th frame. For example, the system may be configured to detect the position of the anatomical feature of interest in at most every second frame of the imaging sensor data.

In various examples, the anatomical feature of interest is detected using a software algorithm, which may be based on a machine-learning model. The effort required by said software algorithm may be reduced, and the accuracy may be improved, by limiting the imaging sensor data to the area of interest. For example, the system may be configured to locate the area of interest within the imaging sensor data. The system may be configured to determine the anatomical feature of interest within a portion of the imaging sensor data representing the area of interest.

Within the area of interest, different anatomical features may be identified by a (machine-learning based) algorithm, and the anatomical feature of interest may be selected among the identified anatomical features. For example, the system may be configured to perform image segmentation on at least the portion of the imaging sensor data representing the area of interest to determine at least one feature present within the portion of the imaging sensor data representing the area of interest. Additionally, or alternatively, the system may be configured to perform object detection on at least the portion of the imaging sensor data representing the area of interest to identify at least one feature present within the portion of the imaging sensor data representing the area of interest. The system may be configured to determine the anatomical feature of interest based on the at least one feature present within the portion of the imaging sensor data representing the area of interest. For example, an existing image segmentation machine-learning model or object detection machine-learning model may be employed for determining the anatomical features present in the area of interest, among which the anatomical feature of interest may be selected.

There are various features that are of potentially of interest to a surgeon, e.g., during brain surgery. For example, the system may be configured to perform the object detection to identify at least one of a blood vessel, branching points of a blood vessel, a bleeding, and a tumor within at least the portion of the imaging sensor data representing the area of interest.

Additionally, non-anatomical features may also be detected and used to define a non-anatomical feature of interest. For example, the system may be configured to perform the object detection to identify at least one of a clip and a stitching as non-anatomical feature of interest. The system may be configured to detect a position of the non-anatomical feature of interest within the imaging sensor data. The system may be configured to perform the autofocus functionality of the microscope further based on the position of the non-anatomical feature of interest. In some cases, non-anatomical features, such as a clip or a stitching, are placed right at the portion of the surgical site the surgeon intends to operate on, making them useful targets with respect to the autofocus functionality.

In some examples, the anatomical feature of interest may be part of a larger anatomical feature. For example, the anatomical feature may be a branch in a blood vessel, which is part of the larger blood vessel. This larger anatomical feature may thus be subdivided into smaller anatomical features, with neighboring anatomical features delimiting the extent of a given anatomical feature within the larger anatomical feature. For example, the system may be configured to determine an extent of the anatomical feature of interest based on an extent of one or more features located adjacent to the feature the anatomical feature of interest is based on.

In some examples, the area of interest might not be definable by a single anatomical feature of interest but may relate to an area between features or to a group of features. For example, the system may be configured to, if the area of interest indicated by the user relates to two or more features or to an area between two or more features, to determine the anatomical feature of interest based on the two or more features, and to determine the position of the anatomical feature of interest based on the positions of the two or more features.

In this case, the features delimiting the area of interest may even be outside the area of interest. Accordingly, the system may be configured to, if the area of interest indicated by the user relates to an area between two or more features, select the two or more features from features located inside the area of interest and features located outside the area of interest.

There are various options for the user to specify the area of interest. For example, the system may be configured to detect a pointer operated by the user within the imaging sensor data to determine the area of interest. Alternatively, or additionally, the system may be configured to determine the area of interest based on a user input signal obtained via a user interface of the surgical microscope system. A pointer or user interface enables a very precise specification of the area of interest.

Alternatively, or additionally, the system is configured to determine the area of interest based on a voice description of an anatomical feature obtained via a voice command system of the surgical microscope system. This way, the area of interest may be defined without the surgeon having to move the instruments away from the surgical site.

The previously listed options allow the surgeon to explicitly define the area of interest. In some examples, however, the area of interest may be defined implicitly, e.g., by deducing the area of interest from the actions of the surgeon. For example, the system may be configured to determine a portion of a surgical site being operated on by the user within the imaging sensor data to determine the area of interest. Alternatively, or additionally, the system may be configured to determine the area of interest using a gaze tracking mechanism. Alternatively, or additionally, the system may be configured to determine the area of interest from a predetermined image area (preferably in the center of the field of view) after the user finishes aligning the field of view. In these cases, the user does not have to explicitly define the area of interest, thus decreasing the effort required by the surgeon.

In some examples, some amount of image processing may be performed to facilitate the definition of the area of interest. For example, the system may be configured to perform image segmentation and/or object detection to determine a plurality of features within the imaging sensor data. The system may be configured to determine a visual representation of the plurality of features and to provide a display signal comprising the visual representation to a display device of the surgical microscope system. The system may be configured to obtain the input of the user in response to the visual representation of the plurality of features. By providing the visual representation of the plurality of features, the user can select among the detected features to define the area of interest.

Surgical microscope systems are often adapted to detect fluorescence emissions that are emitted by a fluorophore that is injected into a blood vessel or tissue. These fluorescence emissions may be used to clearly identify the anatomical feature of interest. Also, additional imaging information such as information gained from multispectral imaging may be used to clearly identify the anatomical feature of interest. For example, the imaging sensor data may comprise a first component with color imaging data and a second component with at least one of hyperspectral imaging data, multispectral imaging data, fluorescence imaging data. The system may be configured to determine the anatomical feature of interest and/or to detect the position of the anatomical feature of interest at least based on the second component.

The determined anatomical (or non-anatomical) feature of interest may be highlighted and presented to the user, e.g., so the user can be assured that the "right" feature is used for the autofocus functionality. For example, the system may be configured to generate a digital view based on the imaging sensor data. The system may be configured to highlight the area of interest and/or the anatomical (or non-anatomical) feature of interest within the digital vie. The system may be configured to provide a display signal comprising the digital view to a display device of the surgical microscope system

Various examples relate to a surgical microscope system comprising a microscope with an optical imaging sensor and the system presented above.

Various examples relate to a corresponding method for a microscope of a surgical microscope system. The method comprises obtaining imaging sensor data from at least one optical imaging sensor of the microscope. The method comprises determining information on an area of interest of a user of the surgical microscope system based on an input of the user. The method comprises determining an anatomical feature of interest within the area of interest. The method comprises detecting a position of the anatomical feature of interest within the imaging sensor data. The method comprises triggering an autofocus functionality of the microscope to focus on the position of the anatomical feature of interest.

Various examples relate to a corresponding computer program with a program code for performing the above method when the computer program is executed on a processor.

### Short description of the Figures

Some examples of apparatuses and/or methods will be described in the following by way of example only, and with reference to the accompanying figures, in which
- Fig. 1a: shows a block diagram of an example of a system for a microscope of a surgical microscope system;
- Fig. 1b: shows a schematic diagram of an example of a surgical microscope system;
- Figs. 1c to 1e: show illustrations of anatomical features of interest relating to blood vessels of a brain;
- Fig. 2: shows a flow chart of an example of a method for a microscope of a surgical microscope system;
- Fig. 3: shows a schematic diagram of an example of a digital view on a surgical site of a brain; and
- Fig. 4: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

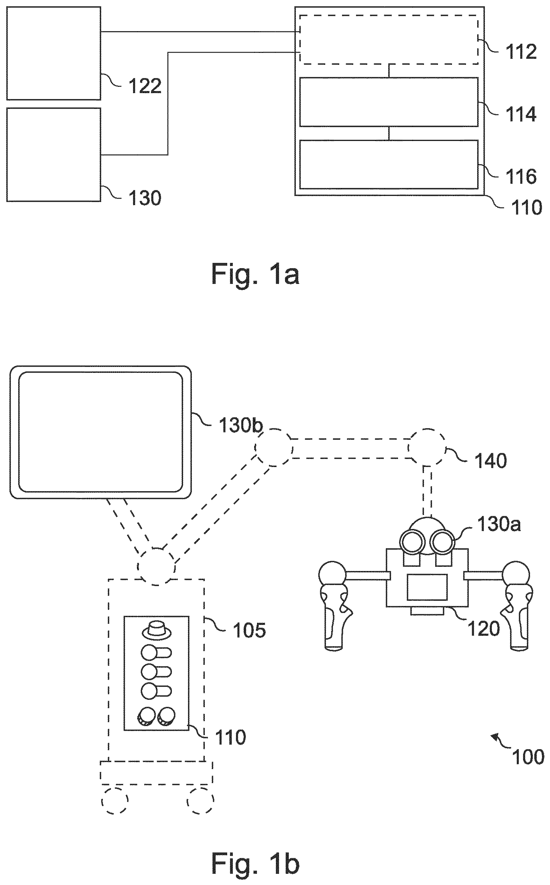

Fig. 1a shows a block diagram of an example of a system 110 for a microscope 120 of a surgical microscope system 100 (shown in more detail in Fig. 1b). The surgical microscope system 100 comprises the microscope 120, which is a digital microscope, i.e., it comprises an optical imaging sensor 122, which is coupled with the system 110. In general, a microscope, such as the microscope 120, is an optical instrument that is suitable for examining objects that are too small to be examined by the human eye (alone). For example, a microscope may provide an optical magnification of a sample. In modern microscopes, the optical magnification is often provided for a camera or an imaging sensor, such as the optical imaging sensor 122 of the microscope 120. The microscope 120 may further comprise one or more optical magnification components that are used to magnify a view on the sample, such as an objective (i.e., lens).

Beside the optical components that are part of the microscope 120, the surgical microscope system 100 further comprises the system 110, which is a computer system. The system comprises one or more processors 114 and one or more storage devices 116. Optionally, the system may further comprise one or more interfaces 112. As shown in Fig. 1a, the one or more processors 114 are coupled with the one or more storage devices 116 and the (optional) one or more interfaces. In general, the functionality of the system is provided by the one or more processors 114, in conjunction with the one or more interfaces 112 (for exchanging information, e.g., with the optical imaging sensor 122 of the microscope or with a display device 130 of the surgical microscope system) and/or with the one or more storage devices 116 (for storing and/or retrieving information). The system 110 is configured to obtain imaging sensor data from the at least one optical imaging sensor 122 of the microscope. The system 110 is configured to determine information on an area of interest of a user of the surgical microscope system based on an input of the user. The system 110 is configured to determine an anatomical feature of interest within the area of interest. The system 110 is configured to detect a position of the anatomical feature of interest within the imaging sensor data. The system 110 is configured to trigger an autofocus functionality of the microscope to focus on the position of the anatomical feature of interest. As is evident, the system 110 is a system for processing imaging sensor data in the surgical microscope system 100 and/or for controlling the microscope 120 and/or other components of the surgical microscope system 100.

In general, a microscope system, such as the surgical microscope system 100, is a system that comprises a microscope 120 and additional components, which are operated together with the microscope, such as the system 110 (which is a computer system being adapted to control the surgical microscope system, and, for example, process imaging sensor data of the microscope), and additional sensors, displays etc.

There are a variety of different types of microscopes. If the microscope is used in the medical or biological fields, the object being viewed through the microscope may be a sample of organic tissue, e.g., arranged within a petri dish or present in a part of a body of a patient. In the present case, the microscope 120 is a microscope of a surgical microscope system, i.e., a microscope that is to be used during a surgical procedure, such as an oncological surgical procedure or during tumor surgery. Accordingly, the object being viewed through the microscope, and shown in the composite image, may be a sample of organic tissue of a patient, and may be in particular be the surgical site that the surgeon operates on during the surgical procedure. In the following, the object to be imaged, i.e., the surgical site, is assumed to be a surgical site of a brain during the course of neurosurgery. However, the proposed concept is also suitable for other types of surgery, such as cardiac surgery or ophthalmology.

Fig. 1b shows a schematic diagram of an example of a surgical microscope system 100 comprising the system 110 and the microscope 120 (with the optical imaging sensor 122). The surgical microscope system 100 shown in Fig. 1b comprises a number of optional components, such as a base unit 105 (comprising the system 110) with a (rolling) stand, ocular displays 130a that are arranged at the microscope 120, an auxiliary display 130b that is arranged at the base unit 105, and a (robotic or manual) arm 140 which holds the microscope 120 in place, and which is coupled to the base unit 105 and to the microscope 120. In general, these optional and non-optional components may be coupled to the system 110 which may be configured to control and/or interact with the respective components.

The proposed concept is based on two main components - obtaining the user input to define the area of interest and using the user-specified area of interest to determine the anatomical feature of interest, which is then tracked so the autofocus can be applied on the anatomical feature of interest even if the field of view changes or if the feature of interest is moved.

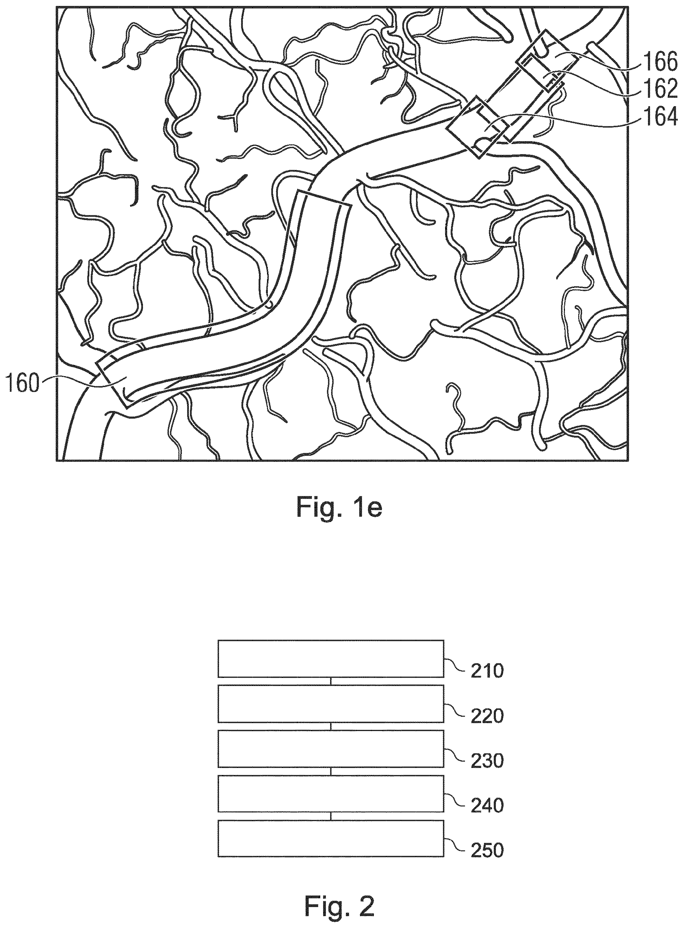

The proposed concept starts by determining the area of interest based on the user input. There are various options for performing this task. For example, the user may use a pointer, such as a finger, a surgical instrument, or a dedicated pointer device (e.g., with an active visual marker such as a light, a passive visual marker such as a two-dimensional code) and point at, or encircle, one or more anatomical features to indicate the area of interest (with the area of interest surrounding the point or circle being indicated by the user). The pointing and/or encircling may be detected using a machine-learning model being trained to determine pointing and/or encircling actions in imaging sensor data or using a deterministic algorithm that is configured to determine the pointing or encircling action based on the imaging sensor data (e.g., based on the active or passive visual marker or by performing object detection on the tip of the finger). The system may be configured to detect the pointer operated by the user within the imaging sensor data to determine the area of interest. For example, the system may be configured to, if the user points at a (single) position in the field of view, determine the area of interest around the (single) point. For example, if the user points at a feature (e.g., an anatomical or non-anatomical feature), the area of interest may surround said feature. The system may be configured to, if the user encircles an area within the field of view, determine the area of interest to coincide with the area being encircled by the user. For example, if the user encircles a feature or set of mutually adjacent features, the area of interest may surround said feature or set of features. The system may be configured to, if the user points at multiple positions, determine, whether the points intersect with the same anatomical feature, and then set the area of interest around said (portion) of the anatomical feature. For example, if the user points multiple times along an elongated blood vessel (e.g., as shown in Fig. 1e), the area of interest may surround a portion of the blood vessel that was pointed at by the surgeon.

A similar approach may be taken if the user defines the area of interest via a user interface, such as a touch screen of the surgical microscope system. For example, the system may be configured to determine the area of interest based on a user input signal obtained via the user interface of the surgical microscope system. Again, the user may use a single or multiple points or a circle to define the area of interest.

To support the selection process, the imaging sensor data may be analyzed to determine and distinguish features in the imaging sensor data, such as anatomical features (e.g., blood vessels, tumors, branches, portions of tissue etc.) or non-anatomical features (e.g., clips or stitches). For this, one or both of the following machine-learning-based techniques may be used - image segmentation and object detection. The system may be configured to perform image segmentation and/or object detection to determine a plurality of features within the imaging sensor data. In object detection, the location of one or more pre-defined objects (i.e., objects that the respective machine-learning model is trained on) in the imaging sensor data is output by a machine-learning model, along with a classification of the object (if the machine-learning model is trained to detect multiple different types of objects). In general, the location of the one or more pre-defined objects is provided as a bounding box, i.e., a set of positions forming a rectangular shape that surrounds the respective object being detected. In image segmentation, the location of features (i.e., portions of the imaging sensor data that have similar attributes, e.g., that belong to the same object) are output by a machine-learning model. In general, the location of the features is provided as a pixel mask, i.e., the location of pixels that belong to a feature are output on a per-feature basis.

For both object detection and image segmentation, a machine-learning model is used that is trained to perform the respective task. For example, to train the machine-learning model being trained to perform object detection, a plurality or samples of imaging sensor data may be provided as training input samples, and a corresponding listing of bounding box coordinates may be provided as desired output of the training, with a supervised learning-based training algorithm being used to perform the training using the plurality of training input samples and corresponding desired output. For example, to train the machine-learning model being trained to perform image segmentation, the plurality or samples of imaging sensor data may be provided as training input samples, and corresponding pixel masks may be provided as desired output of the training, with a supervised learning-based training algorithm being used to perform the training using the plurality of training input samples and corresponding desired output. In some examples, the same machine-learning model may be used to perform both object detection and image segmentation. In this case, the two above-mentioned types of desired output may be used in parallel during the training, with the machine-learning model being trained to output both the bounding boxes and the pixel masks. The machine-learning model might not only be used to support the selection of the area of interest, but also with to determine the anatomical feature of interest, as will be shown at a later stage.

The system may be configured to determine a visual representation of the plurality of features. For example, the system may be configured to determine the visual representation with overlays highlighting and/or delineating the plurality of features, e.g., with an outline around the features (e.g., based on the pixel masks or bounding boxes), and/or with a color overlay being overlaid over the respective features (e.g., based on the pixel masks or bounding boxes). For example, if object detection is being performed, a written description of the feature may be included next to the feature. In addition, a grid may be displayed, which may become useful for a voice-based user interface. For example, the visual representation may be included in a digital view of the surgical site being viewed through the microscope, e.g., overlaid over the digital view. The system may be configured to provide a display signal comprising (digital view with) the visual representation to a display device 130 (e.g., the ocular displays 130a or the auxiliary display 130b) of the surgical microscope system. The user may use the visual representation to select the area of interest, e.g., by pointing at one or multiple of the features or by encircling the features. Accordingly, the system may be configured to obtain the input of the user in response to the visual representation of the plurality of features.

This visual representation may be particularly useful if a voice-based user interface is used to select the area of interest. For example, the system may be configured to determine the area of interest based on a voice description of an anatomical feature obtained via a voice command system of the surgical microscope system. The system may be configured to process the voice description using a voice processing algorithm to associate the anatomical feature being described with one of the plurality of features identified using object detection and/or image segmentation. For example, the system may be configured to, if the voice description comprises a reference to a cell of the grid, select an anatomical feature fitting the description that is shown within the cell of the grid, and determine the area of interest based on the selected anatomical feature. Alternatively, or additionally, a natural language description may be used (e.g., "the largest branch on the lower right side", or "the portion of the blood vessel between the two branches shown on the right side of the view"), and the system may be configured to select the anatomical feature based on the natural language description and determine the area of interest based on the selected anatomical feature.

Above, the area of interest is selected explicitly by the user. Alternatively, the selection of the area of interest may be performed implicitly, with the system interpreting the actions of the user (e.g., the surgeon) to determine the area of interest. For example, the system may be configured to determine a portion of a surgical site being operated on by the user within the imaging sensor data to determine the area of interest. For example, the system may be configured to perform object detection to detect the location of one or more surgical instruments within the imaging sensor data, and to determine the portion of the surgical site being operated on by the user based on the location of the one or more surgical instruments, and to determine the area of interest around the location of the one or more surgical instruments (e.g., around the surgical site-facing side of the one or more surgical instrument). Additionally, or alternatively, the system may be configured to determine the area of interest using a gaze tracking mechanism, e.g., by tracking the gaze of the user through the oculars of the microscope and determining the area of interest to be an area the gaze is focused on for a pre-defined time interval. Alternatively, or additionally, the system may be configured to determine the area of interest from a predetermined image area (within the field of view, e.g., a central area within the field of view covering at least 10% and at most 50% of the field of view) after the user finishes aligning the field of view (i.e., after the user changes the position of the microscope or adjusts the magnification of the microscope.

Once the area of interest is determined, the anatomical feature of interest within the area of interest is selected. In particular, the system may be configured to locate the area of interest within the imaging sensor data (and thus the current field of view), and to determine the anatomical feature of interest within a portion of the imaging sensor data representing the area of interest, e.g., using image processing techniques, such as the aforementioned object detection or image segmentation. In other words, image processing techniques are used to automatically, e.g., without additional user input in addition to the selection of the area of interest, select the anatomical feature of interest.

As outlined above, at least one of the two techniques "object detection" and "image segmentation" may be used to analyze the imaging sensor data and determine features (e.g., anatomical, or non-anatomical features) within the imaging sensor data, with the anatomical feature being selected being among the determined features. For this, either the (optional) previous determination of features across the imaging sensor data may be used, or the object detection or image segmentation may be performed on a subset of the imaging sensor data corresponding to the area of interest (and potentially some surrounding areas, as will become evident in the following). Accordingly, the system may be configured to perform image segmentation on at least the portion of the imaging sensor data representing the area of interest to determine at least one feature present within the portion of the imaging sensor data representing the area of interest, and to determine the anatomical feature of interest based on the at least one feature (e.g., pixel mask) present within (e.g., intersecting with) the portion of the imaging sensor data representing the area of interest. Alternatively, or additionally, the system may be configured to perform object detection on at least the portion of the imaging sensor data representing the area of interest to identify at least one feature (e.g., bounding box) present within (e.g., intersecting with) the portion of the imaging sensor data representing the area of interest, and to determine the anatomical feature of interest based on the identified at least one feature present within the portion of the imaging sensor data representing the area of interest. In other words, if the output of the object detection of image segmentation machine-learning model indicates that one or more features intersect with the area of interest in the imaging sensor data, said feature (or features) may be used as feature(s) of interest. If multiple points are indicated by the user, the feature or combination of features intersecting with all or most of the points may be selected as anatomical feature of interest.

In some examples, the features being used to select the anatomical feature of interest may be restricted to specific groups of features. For example, the system may be configured to perform the object detection to identify at least one of a blood vessel, branching points of a blood vessel, a bleeding, a tumor, a surgical cavity, tissue having a pre-defined color, tissue having a discoloration, an elevated area, and a depressed area within at least the portion of the imaging sensor data representing the area of interest. Accordingly, the machine-learning model being trained to perform object detection may be trained to detect at least one of a blood vessel, branching points of a blood vessel, a bleeding, a tumor, a surgical cavity, tissue having a pre-defined color, tissue having a discoloration, an elevated area, and a depressed area in imaging sensor data. Analogously, the machine-learning model being trained to perform image segmentation may be trained to perform image segmentation for at least one of a blood vessel, branching points of a blood vessel, a bleeding, a tumor, a surgical cavity, tissue having a pre-defined color, tissue having a discoloration, an elevated area, and a depressed area in imaging sensor data. Accordingly, the anatomical feature of interest may be selected based on the output of one or more machine-learning models being trained to output information, such as bounding boxes or pixel masks, representing specific features, such as the aforementioned blood vessel, branching point, bleeding, tumor, surgical cavity, tissue having a predefined color, tissue having a discoloration, elevated area, or depressed area.

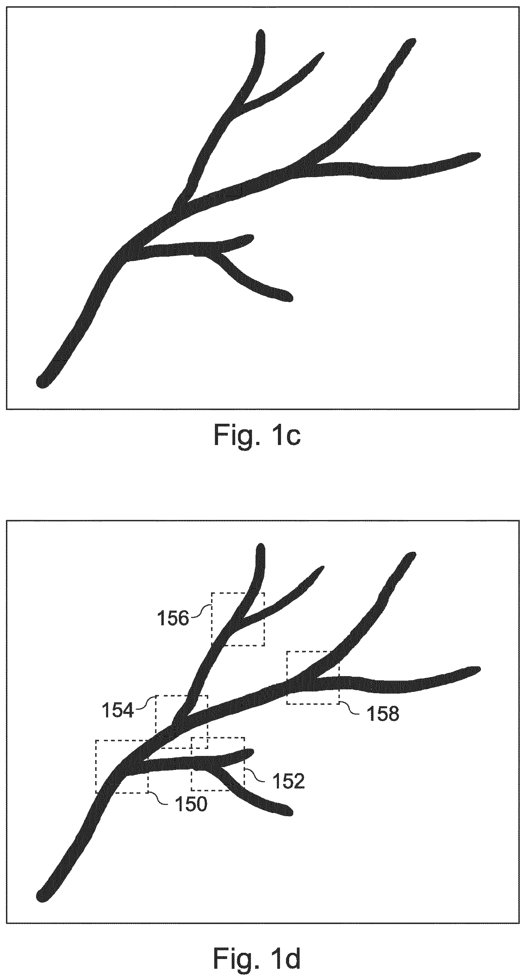

In the following, some examples are given on how the proposed methodology is applied to imaging sensor data collected during neurosurgery. Figs. 1c to 1e show illustrations of anatomical features of interest relating to blood vessels of a brain. For example, the system may identify morphological and color features, such as arteries, veins, and unique shape identifiers on them, e.g., branching. In Fig. 1c, a blood vessel of the brain with multiple branches is shown. In neurosurgery, in many cases, the branches of such blood vessels are of particular interest. Therefore, as shown in Fig. 1d, the system may be configured to use object detection to identify the branching points 150-158 (shown as bounding boxes around the branching points) of the blood vessel. If the area of interest intersects with one of the bounding boxes, said branching point may be selected as anatomical feature of interest. In some examples, the system may be configured to identify low-contrast or non-visible information within the area of interest. For example, if the surgeon points multiple times at a vein, the system may understand that it is about the vein and not the nearby arteries, or if the surgeon clicks on the fully oxygenated blood on the surface of a cavity (micro-bleeding), the system may consider the blood saturation and the shape of this element (micro-bleeding) and thus identify the surgical cavity. In addition, additional image information, such as imaging sensor data from hyperspectral imaging or fluorescence imaging, may be taken into account. For example, the imaging sensor data may comprise a first component with color imaging data (e.g., from white light reflectance imaging) and a second component with at least one of hyperspectral imaging data, multispectral imaging data, and fluorescence imaging data. The second component may comprise information that is not present or not discernible in the first component, e.g., as it is mixed with color information from adjacent wavelength bands, or as the fluorescence emissions are excluded or drowned out from the white light reflectance image (by excluding the respective fluorescence emission wavelength bands from the illumination used to perform the white light reflectance imaging). This additional information may be used to distinguish or select the anatomical feature of interest. Accordingly, the system may be configured to determine the anatomical feature of interest and/or to detect the position of the anatomical feature of interest at least based on the second component, e.g., by using the fluorescence emissions or color information that is isolated in the hyper- or multispectral imaging sensor data to distinguish the anatomical feature of interest from other features visible in the imaging sensor data.

In some examples, the system may also consider the neighboring structures, such as branching of the same vessels, which are not directly within the user defined area, i.e., a certain segment of the may be understood by the algorithm as the vessel part between two characteristic brunches or vessel curvatures. For example, as shown in Fig. 1e, the large curvature 160 may be selected as anatomical feature of interest (being part of the larger blood vessel), e.g., by pointing along the path of the curvature multiple times or by pointing to the center of the curvature. Alternatively, the portion 162 of the blood vessel between the branching points 164; 166 may be selected. Accordingly, the system may be configured to determine an extent of the anatomical feature of interest based on an extent of one or more features located adjacent to the feature the anatomical feature of interest is based on. For example, the system may be configured to limit the extent of the feature of interest based on the features located adjacent, e.g., to limit the portion of the blood vessel 162 being selected as anatomical feature of interest based on the location of two branching points 164; 166 being located adjacent to the portion of the blood vessel.

In some examples, the area between two characteristic features may be deemed to be of interest. In absence of characteristic features, the area of interest, and thus the anatomical feature of interest, may be specified as an area spatially oriented relative to other features even outside the area of interest. For example, the system may be configured to, if the area of interest indicated by the user relates to two or more features or to an area between two or more features, to determine the anatomical feature of interest based on the two or more features, and to determine the position of the anatomical feature of interest based on the positions of the two or more features. Moreover, the system may be configured to, if the area of interest indicated by the user relates to an area between two or more features, select the two or more features from features located inside the area of interest and features located outside the area of interest. For example, the area of interest may be understood by the algorithm as the tissue between two vertically oriented vessels near but outside the user-indicated area of interest. A secondary optical imaging sensor with a wider field of view may also be used for this purpose, i.e., for selecting features outside the area of interest. In some examples, the afore-mentioned image analysis/processing techniques may be used to extract features which are either of low contrast, or invisible, or require quantification. For example, the system may be configured to perform image processing to measure parameters related to vessels/capillaries such as density, branching, and curvature, and use these parameters to segment and identify the anatomical feature of interest. For example, a tissue area with high density of capillaries may be an anatomical feature of interest.

In some examples, the system may also consider foreign objects such as clips or stitches. For example, the system may be configured to perform the object detection to identify at least one of a clip and a stitching as non-anatomical feature of interest. Accordingly, the machine-learning model being trained to perform object detection and/or image segmentation may be trained based on imaging sensor data showing non-anatomical features (as training input samples) and the corresponding bounding box coordinates and/or pixel masks as desired output. The system may be configured to detect a position of the non-anatomical feature of interest within the imaging sensor data, and to perform the autofocus functionality of the microscope further based on the position of the non-anatomical feature of interest. The non-anatomical feature of interest may be used in addition to or instead of the anatomical feature of interest, e.g., the system may support both using the anatomical feature and the non-anatomical feature with respect to the autofocus capability. For example, if the user points at an anatomical feature of interest or the area of interest comprises a (more prominent) anatomical feature, the autofocus functionality may be performed based on the anatomical feature, and if the user points at a non-anatomical feature of interest or the area of interest comprises a (more prominent) non-anatomical feature, the autofocus functionality may be performed based on the non-anatomical feature. In other words, if the user selection (clearly) relates to the non-anatomical feature, the autofocus functionality may be performed based on the non-anatomical feature, else the autofocus functionality may be performed based on the anatomical feature.

Once the anatomical (or non-anatomical) feature of interest is determined, the autofocus functionality is targeted at the feature of interest. In particular, the system is configured to detect the position of the anatomical feature of interest within the imaging sensor data, and to trigger the autofocus functionality of the microscope to focus on the position of the anatomical or non-anatomical feature of interest. In other words, the system may be configured to set the focus point used by the autofocus system to the position of the (anatomical or non-anatomical) feature of interest.

In various examples, the elaborate determination of the feature of interest is performed to enable tracking the feature of interest across frames and reengaging the autofocus functionality once the field of view changes (e.g., because the user has moved the microscope or changed the magnification of the microscope) or once the position of the feature of interest changes. Accordingly, the system may be configured to track the position of the anatomical or non-anatomical feature of interest over a plurality of frames of the imaging sensor data, e.g., by performing the object detection or image segmentation on the plurality of frames and determining a correspondence between features of subsequent frames (e.g., based on the shape of the features indicated by the pixel masks output by the image segmentation). However, since this process is computationally expensive and a difference between subsequent frames is usually low, the tracking may be performed at a lower frame rate than the frame rate of the imaging sensor data. The system may thus be configured to detect the position of the anatomical feature of interest in at most every second frame (e.g., every n-th frame, with *n* ∈ {2, 3, 4, 6, 10, 12,15, 24, 30, 45, 60}) of the imaging sensor data. The system may be configured to trigger the autofocus functionality if the position of the anatomical or non-anatomical feature of interest shifts relative to a field of view of the imaging sensor data (e.g., as the field of view shifts or as the anatomical feature is moved) for at least a pre-defined time interval, e.g., for at least 1, or at least 2, or at least 5, or at least 10 seconds. In other words, the autofocus functionality may be reengaged based on the position of the anatomical or non-anatomical feature of interest if the position of the anatomical or non-anatomical feature of interest non only temporarily changes relative to the field of view. The system may be configured to detect the feature of interest shifting relative to the field of view, e.g., by comparing the position of the feature of interest in successive frames.

In some examples, the feature of interest may change during the surgical procedure. For example, if the feature of interest is a tumor, the tumor may be removed, if the feature of interest is a blood vessel, the blood vessel may be deformed, if the feature of interest is a crossing of two blood vessels, and one of the blood vessels is removed. In such cases, the anatomical feature of interest may be updated and/or redefined. In other words, the system may be configured to repeat determining the anatomical feature of interest if the previously determined feature of interest is removed or deformed. In this case, the area surrounding the previous anatomical feature of interest may be used as area of interest, and the new anatomical feature of interest may be determined within said area of interest. For example, if the anatomical feature of interest is a vessel, and at some point, the vessel is deformed, the new deformed shape may be used in the definition of the anatomical feature of interest. Alternatively, or additionally, a different type of dynamic the anatomical feature of interest definition may be used. For example, the anatomical feature of interest may start as the point where an artery is crossing with a vein, and during the operation the vein may be removed from the field of view. In this case, the anatomical feature of interest may be redefined dynamically as the artery part after a specific branching. In some examples, a smart-dynamic the anatomical feature of interest adaptation may be performed by considering the area where the surgeon is working on. For example, as the surgeon is digging in the tissue towards a deeper tumor, the algorithm may follow the "point of action".

The anatomical or non-anatomical feature of interest being selected, and thus the point being used with respect to the autofocus functionality, may be communicated to the user of the surgical microscope system (e.g., to the surgeon). As outlined above, a digital view of the surgical site may be generated based on the imaging sensor data, showing the surgical site together with additional information, e.g., settings of the surgical microscope system etc. Accordingly, the system may be configured to generate the digital view based on the imaging sensor data. In addition, the selected anatomical or non-anatomical feature of interest may be highlighted, e.g., by showing a color overlay over the anatomical or non-anatomical feature of interest, or by showing a visual indicator representing the focus point. The system may be configured to highlight the area of interest (e.g., as an outline) and/or the feature of interest (e.g., as another outline, color overlay or visual indicator representing the focus point) within the digital view, e.g., by adding an overlay with the outline(s), color overlay or visual indicator. The system may be configured to provide the display signal comprising the digital view to the display device 130 of the surgical microscope system.

In various examples, a visual representation of the plurality of features and/or a digital view of the surgical site are generated and provided to a display 130 of the surgical microscope system as part of a display signal. The visual representation or digital view may be viewed by the user, e.g., the surgeon, of the surgical microscope system. For this purpose, the display signal may be provided to the display, e.g., the auxiliary display 130b or the ocular displays 130a, of the microscope system. Accordingly, the system may be configured to generate the display signal for the display device 130 of the microscope system, the display signal being based on the digital view or visual representation. For example, the display signal may be a signal for driving (e.g., controlling) the display device 130. For example, the display signal may comprise video data and/or control instructions for driving the display. For example, the display signal may be provided via one of the one or more interfaces 112 of the system. Accordingly, the system 110 may comprise a video interface 112 that is suitable for providing the display signal to the display 130 of the microscope system 100.

In the proposed microscope system, at least one optical imaging sensor is used to provide the imaging sensor data. Accordingly, the optical imaging sensor 122 is configured to generate the imaging sensor data. For example, the at least one optical imaging sensor 122 of the microscope 120 may comprise or be an APS (Active Pixel Sensor) - or a CCD (Charge-Coupled-Device)-based imaging sensor 122. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging. The processing system 110 may be configured to obtain (i.e., receive or read out) the imaging sensor data from the optical imaging sensor. The imaging sensor data may be obtained by receiving the imaging sensor data from the optical imaging sensor (e.g., via the interface 112), by reading the imaging sensor data out from a memory of the optical imaging sensor (e.g., via the interface 112), or by reading the imaging sensor data from a storage device 116 of the system 110, e.g., after the imaging sensor data has been written to the storage device 116 by the optical imaging sensor or by another system or processor.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components.

Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the surgical microscope system are mentioned in connection with the proposed concept, or one or more examples described above or below (e.g., Fig. 2 to 4). The surgical microscope system may comprise one or more additional optional features corresponding to one or more aspects of the proposed concept, or one or more examples described above or below.

Fig. 2 shows a flow chart of an example of a corresponding method for a microscope of a surgical microscope system. The method comprises obtaining 210 imaging sensor data from at least one optical imaging sensor of the microscope. The method comprises determining 220 information on an area of interest of a user of the surgical microscope system based on an input of the user. The method comprises determining 230 an anatomical feature of interest within the area of interest. The method comprises detecting 240 a position of the anatomical feature of interest within the imaging sensor data. The method comprises triggering 250 an autofocus functionality of the microscope to focus on the position of the anatomical feature of interest.

For example, the method may be implemented by the surgical microscope system introduced in connection with one of the Figs. 1a to 1e. Features introduced in connection with the surgical microscope system of Figs. 1a to 1e may likewise be included in the corresponding method.

More details and aspects of the method are mentioned in connection with the proposed concept, or one or more examples described above or below (e.g., Fig. 1a to 1e, 3 to 4). The method may comprise one or more additional optional features corresponding to one or more aspects of the proposed concept, or one or more examples described above or below.

Various examples of the present disclosure relate to a concept for an adaptive autofocus.

In general, the surgeon needs to manually focus the region of interest every time they move the microscope. The proposed concept provides an auto focus of the region of interest, which is manually defined (only once) by the surgeon. Thus, there is no need to spend time adjusting the focus each time the surgeon moves the microscope. Consequently, the region of interest may always be on focus.

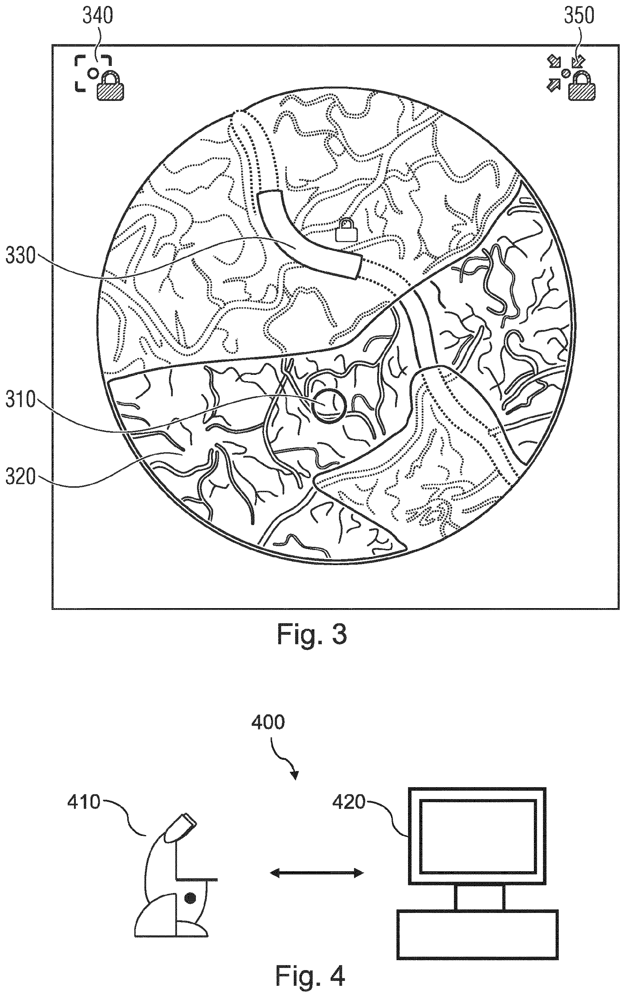

Fig. 3 shows a schematic diagram of an example of a digital view on a surgical site of a brain. Fig. 3 shows the current focus point 310, which was set before the focus point was adjusted based on the determined feature of interest. Based on the focus point 3, the region 320 is in focus. Fig. 3 further shows a feature of interest 330, which corresponds to a curved region of a blood vessel. The lock beside the highlighted feature of interest indicates that the autofocus system is locked to this feature of interest. Fig. 3 further shows a first user interface element 340, which can be triggered to lock focus functionality, and a second user interface element 350, which can be triggered for lock to center functionality.

The proposed concept is based on four components: In a first component, the surgeon identifies an area of interest that needs to remain in focus. In a second component, an algorithm converts the local information, i.e., region of interest (ROI), also area of interest, as discussed in connection with Figs. 1a to 2, into a feature of interest (FOI) to be searched within the image. In a third component, the algorithm searches in each frame for the identified FOI and segments the relevant image part as focus ROI. In a fourth component, the focus ROI is communicated to the autofocus function.

In the following, some implementation examples are given for the four fundamental components listed above. For example, with respect to the first component, the surgeon may use a pointing device, e.g., computer mouse, or touchscreen, to define the ROI. Alternatively, or additionally, the surgeon may use an object within the field of view, e.g., use the tip of a surgical tool, or the fingertip. In some examples, the microscope system may employ an eyetracking device, so that the surgeon can look at an image area and thus indicate the ROI. For example, the defined ROI may be defined using a standard (i.e., geometric) shape (e.g., circle or square), or may be defined using a free-form line outlining the desired structure.

In some examples, the user may point or click once or multiple times on the desired feature, e.g., clicking along a vessel. Additionally, or alternatively, the surgeon may use a voice command, e.g., "follow the branching of the big artery at the upper-left side". In some examples, the microscope may segment the image based on different criteria and the surgeon may select one of the segments, e.g., segmentation of vessels, and vessel features (e.g., branching, deformations, color), surgical cavities, colors/discolorations, and elevated areas. For example, the segmentation may (also) use information beyond the information shown in the color image, such as fluorescence, multispectral/hyperspectral images, and preoperative data (e.g., a tumor is known to be deep in the tissue). For example, the segmentation of the image may be done or assisted by the surgical activity of the surgeon, e.g., the area that the surgeon is cutting, touching, manipulating

After the ROI is selected by the surgeon, the system may identify the feature of interest (FOI), i.e., perform the second component of the proposed concept. For example, the system may identify morphological and color features, such as arteries, veins, and unique shape identifiers on them, e.g., branching. The system may identify low-contrast or non-visible information within the ROI. For example, if the surgeon clicks multiple times on a vein, the system may understand that it is about the vein and not the nearby arteries, or if the surgeon clicks on the fully oxygenated blood on the surface of a cavity (micro-bleeding), the algorithm may consider the blood saturation and the shape of this element (micro-bleeding) and thus identify the surgical cavity. In some examples, the system may also consider the neighboring structures, such as branching of the same vessels, which are not directly within the user defined area, i.e., a certain segment of the may be understood by the algorithm as the vessel part between two characteristic brunches or vessel curvatures. In some examples, the system may consider foreign objects such as clips or stitches.

In absence of characteristic features, it may be possible to define the ROI, and consequently the FOI, as an area spatially oriented relative to other features even outside the ROI. For example, the ROI may be understood by the algorithm as the tissue between two vertically oriented vessels near but outside the user-indicated ROI. A secondary camera with a wider Field of View (FoV) may also be used for this purpose. In some examples, image analysis/processing techniques may be used to extract features which are either of low contrast, or invisible, or require quantification. For example, image processing may measure parameters related to vessels/capillaries such as density, branching, and curvature, and use these parameters to segment and identify the FOI. A tissue area with high density of capillaries may be a FOI. In general, a FOI may be described by any one or more features. The result of the FOI identification process may be visualized to ensure the validity of the recognition. The surgeon may confirm, or the surgeon might only take action if it is wrong. For example, the system may indicate the type of features the algorithm uses for the tracking with specific symbols (e.g., an arrow indicating the size of a discolored patch, an arc indicating the curvature of a vessel, or the presence of a fluorescence signal). Thereby the surgeon may be better equipped to control the tracking validity.

In the third component of the system, the algorithm searches for the FOI. For example, the algorithm may search for the FOI using a dynamic FOI description. For example, the FOI may be searched in each frame, but regularly (e.g., every n-th frame), the description of the FOI may be redefined (i.e., updated). For example, when the FOI is a vessel, and at some point, the vessel is deformed, the new deformed shape may be used in the definition. Alternatively, or additionally, a different type of dynamic FOI definition may be used. For example, in addition to the dynamic FOI definition specified above, the different type of FOI may provide the ability to change the way the FOI is defined. For example, a FOI may start as the point where an artery is crossing with a vein, and during the operation the vein may be removed from the FOV. In this case, the FOI may be redefined dynamically as the artery part after a specific branching. In some examples, a smart-dynamic FOI adaptation may be performed by considering the area where the surgeon is working on. For example, as the surgeon is digging in the tissue towards a deeper tumor, the algorithm may follow the "point of action". Similar to the definition of the FOI, the result of the FOI-searching process may be visualized (e.g., by outlining the current ROI being used for autofocusing). This can be done continuously, periodically (e.g., blinking for 1 second every 15 seconds), when there is a change in the parameter (e.g., when the length of an anatomical structure changes), or when the recognized feature is changed (e.g., the algorithm stops tracking the crossing point of vessels, and now tracks a branching). In some examples, the proposed system may indicate the certainty of the algorithm in the identification of FOI. This may allow the surgeon to redefine the ROI/FOI before a crucial part of the operation and thus avoid the greater inconvenience of the autofocus failure at a crucial surgical step.

With respect to the fourth component, i.e., the communication of the ROI to the autofocus subsystem, the following implementation approaches may be used. For example, the focus ROI may be communicated with a time delay to avoid errors due to instant misinterpretation of the FOI by the algorithm. For example, if the FOI is covered by another object (e.g., tissue, tool, gauze), this should not cause the algorithm "going crazy". For example, the communication frequency may be influenced by the certainty/confidence of the FOI-recognition. For example, when the algorithm is certain (i.e., the FOI is found with a high level of certainty), the focus ROI may be updated instantly, but when the certainty is low, it may wait until either the confidence raises or issue a warning. In some examples, the ROI may contain different weights for ROI zones with different confidence. For example, tissue structures that are recognized well may have 90% weight, and at the same time other tissue areas that have a vague recognition (e.g., as the fluorescence signal is weak) may have 30% weight. This means that the autofocus may give priority in focusing the zones with high confidence.

More details and aspects of the concept for adaptive autofocus are mentioned in connection with the proposed concept or one or more examples described above or below (e.g., Fig. 1a to 2, 4). The concept for adaptive autofocus may comprise one or more additional optional features corresponding to one or more aspects of the proposed concept, or one or more examples described above or below.

Some embodiments relate to a microscope comprising a system as described in connection with one or more of the Figs. 1 to 3. Alternatively, a microscope may be part of or connected to a system as described in connection with one or more of the Figs. 1 to 3. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

## Claims

1. A system (110; 420) for a microscope (120; 410) of a surgical microscope system (100; 400), the system (110; 420) comprising one or more processors (114) and one or more storage devices (116), wherein the system (110; 420) is configured to:
obtain imaging sensor data from at least one optical imaging sensor (122) of the microscope (120; 410);
determine information on an area of interest of a user of the surgical microscope system (100; 400) based on an input of the user;
determine an anatomical feature of interest within the area of interest;
detect a position of the anatomical feature of interest within the imaging sensor data; and
trigger an autofocus functionality of the microscope (120; 410) to focus on the position of the anatomical feature of interest over a plurality of frames of the imaging sensor data.

2. The system (110; 420) according to claim 1, wherein the system (110; 420) is configured to trigger the autofocus functionality if the position of the anatomical feature of interest shifts relative to a field of view of the imaging sensor data for at least a predefined time interval.

3. The system (110; 420) according to claim 2, wherein the system (110; 420)is configured to detect the position of the anatomical feature of interest in at most every second frame of the imaging sensor data.

4. The system (110; 420) according to one of the preceding claims, wherein the system (110; 420) is configured to locate the area of interest within the imaging sensor data, and to determine the anatomical feature of interest within a portion of the imaging sensor data representing the area of interest.

5. The system (110; 420) according to claim 4, wherein the system (110; 420) is configured to perform image segmentation on at least the portion of the imaging sensor data representing the area of interest to determine at least one feature present within the portion of the imaging sensor data representing the area of interest, and to determine the anatomical feature of interest based on the at least one feature present within the portion of the imaging sensor data representing the area of interest.

6. The system (110; 420) according to one of the claims 4 or 5, wherein the system (110; 420) is configured to perform object detection on at least the portion of the imaging sensor data representing the area of interest to identify at least one feature present within the portion of the imaging sensor data representing the area of interest, and to determine the anatomical feature of interest based on the identified at least one feature present within the portion of the imaging sensor data representing the area of interest.

7. The system (110; 420) according to claim 6, wherein the system (110; 420) is configured to perform the object detection to identify at least one of a clip and a stitching as non-anatomical feature of interest, to detect a position of the non-anatomical feature of interest within the imaging sensor data, and to perform the autofocus functionality of the microscope (120; 410) further based on the position of the non-anatomical feature of interest.

8. The system (110; 420) according to one of the claims 4 to 7, wherein the system (110; 420) is configured to, if the area of interest indicated by the user relates to two or more features or to an area between two or more features, to determine the anatomical feature of interest based on the two or more features, and to determine the position of the anatomical feature of interest based on the positions of the two or more features.

9. The system (110; 420) according to claim 9, wherein the system (110; 420) is configured to, if the area of interest indicated by the user relates to an area between two or more features, select the two or more features from features located inside the area of interest and features located outside the area of interest.

10. The system (110; 420) according to one of the preceding claims, wherein the system (110; 420) is configured to detect a pointer operated by the user within the imaging sensor data to determine the area of interest,
or wherein the system (110; 420) is configured to determine a portion of a surgical site being operated on by the user within the imaging sensor data to determine the area of interest,
or wherein the system (110; 420) is configured to determine the area of interest using a gaze tracking mechanism,
or wherein the system (110; 420) is configured to determine the area of interest based on a voice description of an anatomical feature obtained via a voice command system of the surgical microscope system (100; 400),
or wherein the system (110; 420) is configured to determine the area of interest based on a user input signal obtained via a user interface (130) of the surgical microscope system (100; 400),
or wherein the system (110; 420) is configured to determine the area of interest from a predetermined image area after the user finishes aligning the field of view.

11. The system (110; 420) according to one of the preceding claims, wherein the system (110; 420) is configured to perform image segmentation and/or object detection to determine a plurality of features within the imaging sensor data, to determine a visual representation of the plurality of features, provide a display signal comprising the visual representation to a display device (130) of the surgical microscope system (100; 400), and to obtain the input of the user in response to the visual representation of the plurality of features

12. The system (110; 420) according to one of the preceding claims, wherein the imaging sensor data comprises a first component with color imaging data and a second component with at least one of hyperspectral imaging data, multispectral imaging data, fluorescence imaging data, wherein the system (110; 420) is configured to determine the anatomical feature of interest and/or to detect the position of the anatomical feature of interest at least based on the second component.

13. A method for a microscope of a surgical microscope system, the method comprising:
obtaining (210) imaging sensor data from at least one optical imaging sensor of the microscope;
determining (220) information on an area of interest of a user of the surgical microscope system based on an input of the user;
determining (230) an anatomical feature of interest within the area of interest;
detecting (240) a position of the anatomical feature of interest within the imaging sensor data; and
triggering (250) an autofocus functionality of the microscope to focus on the position of the anatomical feature of interest over a plurality of frames of the imaging sensor data if the position of the anatomical feature of interest shifts relative to a field of view of the imaging sensor data for at least a pre-defined time interval.

14. A computer program with a program code for performing the method according to claim 13 when the computer program is executed on a processor of a system of claim 1.

## Patentansprüche

1. System (110; 420) für ein Mikroskop (120; 410) eines Operationsmikroskopsystems (100; 400), wobei das System (110; 420) einen oder mehrere Prozessoren (114) und einen oder mehrere Speicher (116) umfasst, wobei das System (110; 420) konfiguriert ist:
Bildsensordaten von mindestens einem optischen Bildsensor (122) des Mikroskops (120; 410) zu erhalten;
Informationen über einen Bereich von Interesse eines Benutzers des Operationsmikroskopsystems (100; 400) basierend auf einer Eingabe des Benutzers zu bestimmen;
ein anatomisches Merkmal von Interesse innerhalb des Bereichs von Interesse zu bestimmen;
eine Position des anatomischen Merkmals von Interesse innerhalb der Bildsensordaten zu detektieren; und
eine Autofokusfunktionalität des Mikroskops (120; 410) auszulösen, um über eine Vielzahl von Einzelbildern der Bildsensordaten auf die Position des anatomischen Merkmals von Interesse zu fokussieren.

2. System (110; 420) nach Anspruch 1, wobei das System (110; 420) konfiguriert ist, die Autofokusfunktionalität auszulösen, wenn sich die Position des anatomischen Merkmals von Interesse relativ zu einem Sichtfeld der Bildsensordaten mindestens für ein vordefiniertes Zeitintervall verschiebt.

3. System (110; 420) nach Anspruch 2, wobei das System (110; 420) konfiguriert ist, die Position des anatomischen Merkmals von Interesse in höchstens jedem zweiten Einzelbild der Bildsensordaten zu detektieren.

4. System (110; 420) nach einem der vorstehenden Ansprüche, wobei das System (110; 420) konfiguriert ist, den Bereich von Interesse innerhalb der Bildsensordaten zu lokalisieren und das anatomische Merkmal von Interesse innerhalb eines Teils der Bildsensordaten, der den Bereich von Interesse darstellt, zu bestimmen.

5. System (110; 420) nach Anspruch 4, wobei das System (110; 420) konfiguriert ist, eine Bildsegmentierung an mindestens dem Teil der Bildsensordaten, der den Bereich von Interesse darstellt, durchzuführen, um mindestens ein innerhalb des Teils der Bildsensordaten, der den Bereich von Interesse darstellt, vorhandenes Merkmal zu bestimmen, und das anatomische Merkmal von Interesse basierend auf dem mindestens einen innerhalb des Teils der Bildsensordaten, der den Bereich von Interesse darstellt, vorhandenen Merkmal zu bestimmen.

6. System (110; 420) nach einem der Ansprüche 4 oder 5, wobei das System (110; 420) konfiguriert ist, eine Objekterkennung an mindestens dem Teil der Bildsensordaten, der den Bereich von Interesse darstellt, durchzuführen, um mindestens ein innerhalb des Teils der Bildsensordaten, der den Bereich von Interesse darstellt, vorhandenes Merkmal zu identifizieren, und das anatomische Merkmal von Interesse basierend auf dem identifizierten mindestens einen innerhalb des Teils der Bildsensordaten, der den Bereich von Interesse darstellt, vorhandenen Merkmal zu bestimmen.

7. System (110; 420) nach Anspruch 6, wobei das System (110; 420) konfiguriert ist, die Objekterkennung durchzuführen, um mindestens eines von einem Clip und einer Naht als nichtanatomisches Merkmal von Interesse zu identifizieren, eine Position des nichtanatomischen Merkmals von Interesse innerhalb der Bildsensordaten zu detektieren und die Autofokusfunktionalität des Mikroskops (120; 410) ferner basierend auf der Position des nichtanatomischen Merkmals von Interesse auszuführen.

8. System (110; 420) nach einem der Ansprüche 4 bis 7, wobei das System (110; 420) konfiguriert ist, wenn sich der von dem Benutzer angegebene Bereich von Interesse auf zwei oder mehr Merkmale oder auf einen Bereich zwischen zwei oder mehr Merkmalen bezieht, das anatomische Merkmal von Interesse basierend auf den zwei oder mehr Merkmalen zu bestimmen und die Position des anatomischen Merkmals von Interesse basierend auf den Positionen der zwei oder mehr Merkmale zu bestimmen.

9. System (110; 420) nach Anspruch 9, wobei das System (110; 420) konfiguriert ist, wenn sich der von dem Benutzer angegebene Bereich von Interesse auf einen Bereich zwischen zwei oder mehr Merkmalen bezieht, die zwei oder mehr Merkmale aus innerhalb des Bereichs von Interesse angeordneten Merkmalen und außerhalb des Bereichs von Interesse angeordneten Merkmalen auszuwählen.

10. System (110; 420) nach einem der vorstehenden Ansprüche, wobei das System (110; 420) konfiguriert ist, einen von dem Benutzer betätigten Zeiger innerhalb der Bildsensordaten zu detektieren, um den Bereich von Interesse zu bestimmen,
oder wobei das System (110; 420) konfiguriert ist, einen Teil einer Operationsstelle, an der der Benutzer operiert, innerhalb der Bildsensordaten zu bestimmen, um den Bereich von Interesse zu bestimmen,
oder wobei das System (110; 420) konfiguriert ist, den Bereich von Interesse unter Verwendung eines Blickverfolgungsmechanismus zu bestimmen,
oder wobei das System (110; 420) konfiguriert ist, den Bereich von Interesse basierend auf einer über ein Sprachbefehlssystem des Operationsmikroskopsystems (100; 400) erhaltenen sprachlichen Beschreibung eines anatomischen Merkmals zu bestimmen,
oder wobei das System (110; 420) konfiguriert ist, den Bereich von Interesse basierend auf einem über eine Benutzeroberfläche (130) des Operationsmikroskopsystems (100; 400) erhaltenen Benutzereingabesignal zu bestimmen,
oder wobei das System (110; 420) konfiguriert ist, den Bereich von Interesse aus einem vorbestimmten Bildbereich zu bestimmen, nachdem der Benutzer das Ausrichten des Sichtfelds beendet.

11. System (110; 420) nach einem der vorstehenden Ansprüche, wobei das System (110; 420) konfiguriert ist, eine Bildsegmentierung und/oder Objekterkennung durchzuführen, um eine Vielzahl von Merkmalen innerhalb der Bildsensordaten zu bestimmen, eine visuelle Darstellung der Vielzahl von Merkmalen zu bestimmen, ein Anzeigesignal, das die visuelle Darstellung umfasst, an eine Anzeigevorrichtung (130) des Operationsmikroskopsystems (100; 400) bereitzustellen und die Eingabe des Benutzers als Reaktion auf die visuelle Darstellung der Vielzahl von Merkmalen zu erhalten

12. System (110; 420) nach einem der vorstehenden Ansprüche, wobei die Bildsensordaten eine erste Komponente mit Farbbilddaten und eine zweite Komponente mit mindestens einem der hyperspektralen Bilddaten, multispektralen Bilddaten, Fluoreszenzbilddaten umfassen, wobei das System (110; 420) konfiguriert ist, das anatomische Merkmal von Interesse zu bestimmen und/oder die Position des anatomischen Merkmals von Interesse mindestens basierend auf der zweiten Komponente zu detektieren.

13. Verfahren für ein Mikroskop eines Operationsmikroskopsystems, wobei das Verfahren Folgendes umfasst:
Erhalten (210) von Bildsensordaten von mindestens einem optischen Bildsensor des Mikroskops;
Bestimmen (220) von Informationen über einen Bereich von Interesse eines Benutzers des Operationsmikroskopsystems basierend auf einer Eingabe des Benutzers;
Bestimmen (230) eines anatomischen Merkmals von Interesse innerhalb des Bereichs von Interesse;
Detektieren (240) einer Position des anatomischen Merkmals von Interesse innerhalb der Bildsensordaten; und
Auslösen (250) einer Autofokusfunktionalität des Mikroskops, um über eine Vielzahl von Einzelbildern der Bildsensordaten auf die Position des anatomischen Merkmals von Interesse zu fokussieren, wenn sich die Position des anatomischen Merkmals von Interesse relativ zu einem Sichtfeld der Bildsensordaten mindestens für ein vordefiniertes Zeitintervall verschiebt.

14. Computerprogramm mit einem Programmcode zur Durchführung des Verfahrens nach Anspruch 13, wenn das Computerprogramm auf einem Prozessor eines Systems nach Anspruch 1 ausgeführt wird.

## Revendications

1. Système (110 ; 420) pour un microscope (120 ; 410) d'un système de microscope chirurgical (100 ; 400), le système (110 ; 420) comprenant un ou plusieurs processeurs (114) et un ou plusieurs dispositifs de stockage (116), dans lequel le système (110 ; 420) est configuré pour :
obtenir des données de capteur d'imagerie à partir d'au moins un capteur d'imagerie optique (122) du microscope (120 ; 410) ;
déterminer des informations sur une région d'intérêt d'un utilisateur du système de microscope chirurgical (100 ; 400) sur la base d'une entrée de l'utilisateur ;
déterminer un élément anatomique d'intérêt à l'intérieur de la région d'intérêt ;
détecter une position de l'élément anatomique d'intérêt à l'intérieur des données de capteur d'imagerie ; et
déclencher une fonction de mise au point automatique du microscope (120 ; 410) pour effectuer la mise au point sur la position de l'élément anatomique d'intérêt sur une pluralité d'images des données de capteur d'imagerie.

2. Système (110 ; 420) selon la revendication 1, dans lequel le système (110 ; 420) est configuré pour déclencher la fonction de mise au point automatique si la position de l'élément anatomique d'intérêt se déplace par rapport à un champ de vision des données de capteur d'imagerie pendant au moins un intervalle de temps prédéfini.

3. Système (110 ; 420) selon la revendication 2, dans lequel le système (110 ; 420) est configuré pour détecter la position de l'élément anatomique d'intérêt dans au plus chaque deuxième image des données de capteur d'imagerie.

4. Système (110 ; 420) selon l'une des revendications précédentes, dans lequel le système (110 ; 420) est configuré pour localiser la région d'intérêt à l'intérieur des données de capteur d'imagerie, et pour déterminer l'élément anatomique d'intérêt à l'intérieur d'une portion des données de capteur d'imagerie représentant la région d'intérêt.

5. Système (110 ; 420) selon la revendication 4, dans lequel le système (110 ; 420) est configuré pour effectuer une segmentation d'image sur au moins la portion des données de capteur d'imagerie représentant la région d'intérêt afin de déterminer au moins un élément présent à l'intérieur de la portion des données de capteur d'imagerie représentant la région d'intérêt, et pour déterminer l'élément anatomique d'intérêt sur la base de l'au moins un élément présent à l'intérieur de la portion des données de capteur d'imagerie représentant la région d'intérêt.

6. Système (110 ; 420) selon l'une des revendications 4 ou 5, dans lequel le système (110 ; 420) est configuré pour effectuer une détection d'objets sur au moins la portion des données de capteur d'imagerie représentant la région d'intérêt afin d'identifier au moins un élément présent à l'intérieur de la portion des données de capteur d'imagerie représentant la région d'intérêt, et pour déterminer l'élément anatomique d'intérêt sur la base de l'au moins un élément identifié présent à l'intérieur de la portion des données de capteur d'imagerie représentant la région d'intérêt.

7. Système (110 ; 420) selon la revendication 6, dans lequel le système (110 ; 420) est configuré pour effectuer la détection d'objets afin d'identifier au moins un parmi un clip et une suture en tant qu'élément non anatomique d'intérêt, pour détecter une position de l'élément non anatomique d'intérêt dans les données de capteur d'imagerie, et pour mettre en œuvre la fonction de mise au point automatique du microscope (120 ; 410) en outre sur la base de la position de l'élément non anatomique d'intérêt.

8. Système (110 ; 420) selon l'une des revendications 4 à 7, dans lequel le système (110 ; 420) est configuré pour, si la région d'intérêt indiquée par l'utilisateur se rapporte à deux éléments ou plus ou à une région entre deux éléments ou plus, déterminer l'élément anatomique d'intérêt sur la base des deux éléments ou plus, et déterminer la position de l'élément anatomique d'intérêt sur la base des positions des deux éléments ou plus.

9. Système (110 ; 420) selon la revendication 9, dans lequel le système (110 ; 420) est configuré pour, si la région d'intérêt indiquée par l'utilisateur se rapporte à une région entre deux éléments ou plus, sélectionner les deux éléments ou plus parmi des éléments situés à l'intérieur de la région d'intérêt et des éléments situés à l'extérieur de la région d'intérêt.

10. Système (110 ; 420) selon l'une des revendications précédentes, dans lequel le système (110 ; 420) est configuré pour détecter un pointeur actionné par l'utilisateur à l'intérieur des données de capteur d'imagerie pour déterminer la région d'intérêt, ou dans lequel le système (110 ; 420) est configuré pour déterminer une portion d'un site chirurgical sur lequel l'utilisateur opère à l'intérieur des données de capteur d'imagerie pour déterminer la région d'intérêt,
ou dans lequel le système (110 ; 420) est configuré pour déterminer la région d'intérêt en utilisant un mécanisme de suivi du regard,
ou dans lequel le système (110 ; 420) est configuré pour déterminer la région d'intérêt sur la base d'une description vocale d'un élément anatomique obtenue par l'intermédiaire d'un système de commande vocale du système de microscope chirurgical (100 ; 400),
ou dans lequel le système (110 ; 420) est configuré pour déterminer la région d'intérêt sur la base d'un signal d'entrée utilisateur obtenu par l'intermédiaire d'une interface utilisateur (130) du système de microscope chirurgical (100 ; 400),
ou dans lequel le système (110 ; 420) est configuré pour déterminer la région d'intérêt à partir d'une zone d'image prédéterminée après que l'utilisateur a fini d'aligner le champ de vision.

11. Système (110 ; 420) selon l'une des revendications précédentes, dans lequel le système (110 ; 420) est configuré pour effectuer une segmentation d'image et/ou une détection d'objets pour déterminer une pluralité d'éléments à l'intérieur des données de capteur d'imagerie, pour déterminer une représentation visuelle de la pluralité d'éléments, fournir un signal d'affichage comprenant la représentation visuelle à un dispositif d'affichage (130) du système de microscope chirurgical (100 ; 400), et pour obtenir l'entrée de l'utilisateur en réponse à la représentation visuelle de la pluralité d'éléments

12. Système (110 ; 420) selon l'une des revendications précédentes, dans lequel les données de capteur d'imagerie comprennent une première composante avec des données d'imagerie couleur et une seconde composante avec au moins un parmi des données d'imagerie hyperspectrale, des données d'imagerie multispectrale, des données d'imagerie par fluorescence, dans lequel le système (110 ; 420) est configuré pour déterminer l'élément anatomique d'intérêt et/ou pour détecter la position de l'élément anatomique d'intérêt au moins sur la base de la seconde composante.

13. Procédé pour un microscope d'un système de microscope chirurgical, le procédé comprenant : obtenir (210) des données de capteur d'imagerie à partir d'au moins un capteur d'imagerie optique du microscope ;
déterminer (220) des informations sur une région d'intérêt d'un utilisateur du système de microscope chirurgical sur la base d'une entrée de l'utilisateur ;
déterminer (230) un élément anatomique d'intérêt à l'intérieur de la région d'intérêt ; détecter (240) une position de l'élément anatomique d'intérêt à l'intérieur des données de capteur d'imagerie ; et
déclencher (250) une fonction de mise au point automatique du microscope pour effectuer la mise au point sur la position de l'élément anatomique d'intérêt sur une pluralité d'images des données de capteur d'imagerie si la position de l'élément anatomique d'intérêt se déplace par rapport à un champ de vision des données de capteur d'imagerie pendant au moins un intervalle de temps prédéfini.

14. Programme informatique avec un code de programme pour exécuter le procédé selon la revendication 13 lorsque le programme informatique est exécuté sur un processeur d'un système selon la revendication 1.
